# FASCICULE DE BREVET EUROPEEN

(11) **EP 3 362 133 B1**
(45) Date de publication et mention de la délivrance du brevet: **15.05.2019**
(21) Numéro de dépôt: 16788173.9
(22) Date de dépôt: 05.10.2016
(51) Int. Cl.: A61M 16/22, A62B 17/04, A62B 7/14, A62B 7/02, A62B 7/10, A62B 19/00, B63C 11/24

(54) **CAGOULE DE PROTECTION RESPIRATOIRE**
ATEMSCHUTZHAUBE
RESPIRATORY PROTECTION HOOD

(30) Priorité: 15.10.2015 FR 1559783
(43) Date de publication de la demande: 22.08.2018
(73) Titulaire: L'Air Liquide Société Anonyme pour l'Etude et l'Exploitation des Procédés Georges Claude, 75007 Paris (FR)
(72) Inventeur: MAKHLOUCHE, Rachid, 38000 Grenoble (FR); CAZENAVE, Jean-Michel, 38180 Seyssins (FR); ROUSSIN-BOUCHARD, Xavier, 38112 Meaudre (FR); COHARD, Pierrick, 38490 Les Abrets (FR); GRAZIANI, Vincent, 75007 Paris (FR)
(74) Mandataire: De Cuenca, Emmanuel Jaime
(86) Numéro de dépôt international: PCT/FR2016/052553
(87) Numéro de publication internationale: WO 2017/064393

(56) Documents cités:
- WO-A-90/00421
- FR-A1- 2 582 524
- US-A- 5 724 958

## Description

L'invention concerne une cagoule de protection respiratoire.

L'invention concerne plus particulièrement une cagoule de protection respiratoire comprenant une enveloppe souple étanche destinée à être enfilée sur la tête d'un utilisateur, l'enveloppe souple étant munie d'une fenêtre transparente et comprenant, dans sa partie inférieure, un élément de base rigide de destiné à être disposé autour du cou de l'utilisateur, l'élément de base comprenant un réservoir d'oxygène sous pression muni un orifice de sortie calibré débouchant dans le volume interne de l'enveloppe souple et un dispositif de captation d'au moins une partie du dioxyde de carbone expiré par l'utilisateur, pour épurer le gaz respirable au sein de la cagoule.

Ce type d'équipement est généralement obligatoire à bord des avions de transport de passagers civils ainsi que dans certains avions de transport militaires. On pourra se référer par exemple au document FR2582524A1

L'invention concerne en particulier équipement de protection respiratoire fonctionnant en circuit fermé. Ce type de fonctionnement en circuit fermé permet de minimiser l'encombrement et la masse d'un équipement respiratoire. Pour cela, l'équipement doit comprendre un volume étanche (enveloppe de cagoule) autour de la tête de l'utilisateur dans lequel de l'oxygène est libéré et la concentration de dioxyde de carbone maintenu à une valeur basse déterminée. Typiquement, le pourcentage de dioxyde de carbone doit rester inférieur à 4% (au niveau de la mer).

Pour maintenir ce taux bas de dioxyde de carbone, une solution est de prévoir dans la cagoule un dispositif de captation du dioxyde de carbone, par exemple une cartouche d'absorption de dioxyde de carbone (CO2).

Les cartouches à structure radiale sont connues pour leur efficacité d'épuration et la faible perte de charge qu'elles engendrent. Les cartouches radiales optimisent le temps de résidence du gaz dans la cartouche (et donc l'efficacité de l'épuration). Cependant, ce type de cartouche est généralement très encombrant et peu ergonomique. (cf. par exemple US2010242966A ou US7520280).

En particulier, ces cartouches nécessitent d'avoir un collecteur de gaz et un diffuseur de gaz de sections suffisantes pour limiter la perte de charge. De plus, la nécessité de prévoir un volume de matériau filtrant suffisant peut rapidement conduire à un diamètre extérieur de cartouche conséquent. La longueur des dispositifs connus bien que bénéfique vis-à-vis du temps de résidence du gaz et de la perte de charge, rend l'intégration ergonomique de la cartouche dans un équipement de protection respiratoire plus difficile.

Un but de la présente invention est de pallier tout ou partie des inconvénients de l'art antérieur relevés ci-dessus.

A cette fin, la cagoule selon l'invention, par ailleurs conforme à la définition générique qu'en donne le préambule ci-dessus, est essentiellement caractérisé en ce que le dispositif de captation du dioxyde de carbone comprend une cartouche d'adsorption du dioxyde de carbone à structure radiale comprenant un corps tubulaire s'étendant selon une direction longitudinale et abritant un volume de matériau absorbant du dioxyde de carbone, ladite structure de la cartouche comprenant une tube ajouré disposée autour du volume de matériau absorbant et s'entendant selon la direction longitudinale pour permettre le transite d'air à épurer radialement dans le volume de matériau à absorbant, c'est-à-dire perpendiculairement à la direction longitudinale, et en ce qu'au moins une partie du tube ajouré disposé autour du volume de matériau absorbant communique directement avec le volume interne de la cagoule, c'est-à-dire qu'au moins une partie du tube ajouré n'est pas entouré d'une paroi extérieure supplémentaire pleine délimitant un volume diffuseur autour du tube ajouré.

Par ailleurs, des modes de réalisation de l'invention peuvent comporter l'une ou plusieurs des caractéristiques suivantes :
- le tube ajouré comprend une paroi extérieure ajourée et une paroi intérieure ajourée, le volume de matériau à absorbant étant disposé entre les parois intérieure et extérieure,
- l'air épuré ayant transité dans le volume de matériau absorbant est collecté dans un volume collecteur d'air purifié, au moins une partie du volume collecteur d'air purifié étant délimité par la paroi intérieure du tube ajouré,
- au moins une partie de la surface périphérique du tube ajouré comprend des saillies qui s'étendent radialement vers l'extérieur du tube ajouré par rapport à l'axe longitudinal, pour éviter que l'enveloppe souple ou la peau de l'utilisateur n'obstrue les orifices du tube ajouré,

- les saillies s'étendent radialement par rapport à l'axe longitudinal avec une hauteur comprise entre 1 et 5mm par rapport au reste de la surface périphérique du tube ajouré,
- le tube comprend des saillies réparties le long de la direction longitudinal, les saillies étant espacées les unes des selon la direction longitudinale d'une distance comprise entre 10mm et 50mm
- le tube comprend des saillies réparties sur au moins une partie et de préférence sur sa circonférence,
- le tube comprend des saillies formant des nervures longitudinales parallèles à l'axe longitudinal,
- le tube comprend des saillies formant des nervures transversales perpendiculaires à l'axe longitudinal,
- le tube ajouré comprend une pluralité d'orifice prévus pour le passage de gaz, lesdits orifices ayant des surfaces comprises entre 3,14mm² et 600mm²
- le tube ajouré comprend sur sa surface une pluralité d'orifice de forme circulaire et/ou oblongue et/ou trapézoïdales, lesdits orifices allongés étant parallèles ou perpendiculaires à la direction longitudinale,
- le tube ajouré est composé d'une structure métallique ou plastique grillagée,
- le tube ajouré est composé d'une structure de base comprenant un squelette recouvert d'une feuille ajourée, notamment d'une feuille plastique ajourée fixée sur la structure de base,
- la feuille ajourée est en matériau plastique et est collée ou surmoulée sur la structure de base,
- le tube ajouré est cintré ou rectiligne.

L'invention peut concerner également tout dispositif ou procédé alternatif comprenant toute combinaison des caractéristiques ci-dessus ou ci-dessous.

D'autres particularités et avantages apparaîtront à la lecture de la description ci-après, faite en référence aux figures dans lesquelles :
- la figure 1 représente une vue de face, schématique et partielle et en partie en transparence, illustrant la structure d'un exemple de réalisation possible d'une cagoule selon l'invention,
- la figure 2 représente une vue de dessous d'un détail de la cagoule de la figure 1,
- la figure 3 illustre un exemple de dispositif d'injection de gaz à effet venturi pouvant être mis en oeuvre dans la cagoule selon l'invention,
- la figure 4 représente un vue schématique illustrant l'intégration et le fonctionnement du dispositif d'injection de gaz dans la cagoule selon l'invention,
- la figure 5 représente une vue en coupe, schématique et partielle d'un détail d'une cagoule selon l'invention illustrant la structure d'un dispositif de captation de CO2 selon l'invention,
- la figure 6 représente une vue en perspective d'un détail de la paroi extérieure ajourée d'une cartouche d'absorption de CO2 selon l'invention,
- la figure 7 représente une vue en perspective d'un détail de la paroi intérieure ajourée d'une cartouche d'absorption de CO2 selon l'invention raccordée à un collecteur de sortie,
- la figure 8 représente une vue en perspective d'un détail d'un autre exemple de réalisation possible de la structure de la paroi ajourée d'une cartouche d'absorption de CO2 selon l'invention,
- la figure 9 représente une vue en perspective, schématique et en coupe transversale, d'un détail d'un exemple de structure d'une cartouche d'absorbant de CO2 selon l'invention,
- la figure 10 représente une vue en perspective d'un détail d'un autre exemple de réalisation possible de réalisation de la structure de la paroi ajourée d'une cartouche d'absorption de CO2 selon l'invention.

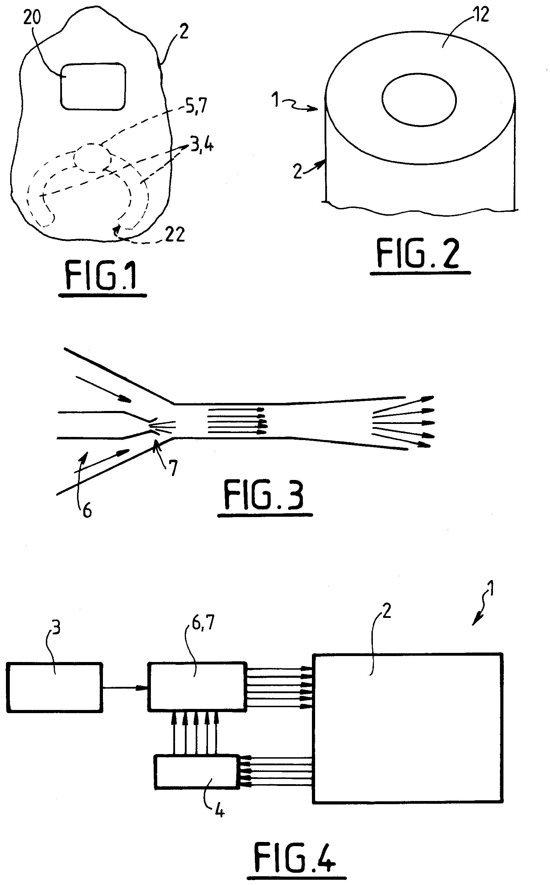

L'exemple non limitatif de cagoule de protection respiratoire représentée à la figure 1 comprend une enveloppe 2 souple étanche destinée à être enfilée sur la tête d'un utilisateur.

De préférence 2 souple est munie d'une fenêtre 20 transparente et comprenant, dans sa partie inférieure, un élément 3, 4 de base rigide de destiné à être disposé autour du cou de l'utilisateur.

L'enveloppe 2 souple étanche est composée par exemple d'un textile anti-feu étanche ou rendu étanche. Par exemple il s'agit d'un textile de fibres synthétiques à faible combustibilité.

Cette enveloppe 2 est prévue pour être enfilée sur la tête de l'utilisateur pour isoler la tête de l'utilisateur du milieu extérieur et pour intégrer les fonctionnalités décrites ci-après. Pour réaliser une étanchéité au niveau du cou de l'utilisateur et comme décrit à la figure 2, la base de l'enveloppe 2 souple peut comporter un diaphragme souple ou une collerette 12 étanche destinée à être montée autour du cou d'un utilisateur. Par exemple, cette collerette 12 inférieure comprend une feuille de plastique percée (par exemple un polymère, notamment un polymère auto-extinguible tel que du Néoprène ou du silicone) pour laisser passer élastiquement la tête de l'utilisateur puis serrer son cou par la suite. L'orifice inférieur pour la tête peut être adapté à tout périmètre de cou compris entre 28 et 42cm par exemple.

L'élément 3, 4 de base comprenant un réservoir 3 d'oxygène sous pression muni un orifice 7 de sortie calibré débouchant dans le volume interne de l'enveloppe 2 souple et un dispositif de captation d'au moins une partie du dioxyde de carbone expiré par l'utilisateur. Le dispositif de captation de CO2 est prévu pour épurer le gaz respirable au sein de la cagoule 2 pour maintenir le taux de CO2 en-dessous d'un seuil déterminé.

Comme schématisé, l'élément 3, 4 de base peut le cas échéant être en au moins deux parties articulées (articulation 5).

Le dispositif de captation du CO2 comprend une cartouche 4 d'adsorption du dioxyde de carbone. La cartouche 4 comprend un corps tubulaire s'étendant selon une direction A longitudinale et abritant un volume 8 de matériau absorbant du dioxyde de carbone. Cette cartouche 4 est à structure radiale. C'est-à-dire que, la structure de la cartouche 4 comprenant une tube 9 ajouré disposée autour du volume 8 de matériau absorbant et s'entendant selon la direction A longitudinale pour permettre le transite d'air à épurer radialement dans le volume 8 de matériau à absorbant. C'est-à-dire que le gaz à épurer traverse la cartouche filtrante perpendiculairement à la direction A longitudinale.

Selon une particularité avantageuse, au moins une partie du tube 9 ajouré disposé autour du volume de matériau absorbant communique directement avec le volume interne de la cagoule 2. C'est-à-dire qu'au moins une partie du tube 9 ajouré (c'est-à-dire la paroi trouée) n'est pas entouré d'une paroi extérieure supplémentaire pleine délimitant un volume diffuseur autour du tube 9 ajouré.

Cette structure améliore la masse, le volume et le fonctionnement de la cagoule et notamment son système de captation de CO2.

Classiquement, les cartouches d'absorption de CO2 radiale comprennent radialement de l'intérieur vers l'extérieur : d'une âme centrale collectant le gaz purifié, une masse de matériau adsorbant le CO2 disposé autour de l'âme centrale et disposée dans une paroi tubulaire ajourée, une paroi pleine extérieure disposée autour de la paroi tubulaire ajourée. Le gaz à purifier entre par une extrémité de la cartouche et vient circuler dans l'espace (appelé « distributeur » ou « diffuseur ») entre la paroi pleine extérieure et la paroi tubulaire ajourée. Le gaz à purifier entre radialement dans le matériau d'adsorption du CO2 (via le tous de la paroi ajourée) et ressort purifié dans l'âme centrale (via des trous). C'est-à-dire que l'âme centrale forme un collecteur de gaz purifié vers une sortie à une extrémité de la cartouche.

Les positions du collecteur de gaz purifié et du distributeur peuvent éventuellement être inter-changées (c'est-à-dire que le gaz entre par l'âme centrale et la cartouche et ressort par la périphérie en transitant radialement de l'intérieur vers l'extérieur au lieu du contraire).

Selon l'invention, la paroi extérieure délimitant le distributeur est omise. Ceci permet de diminuer le volume et la masse du dispositif permettant son intégration dans une cagoule. En particulier, cette structure, sans nuire à l'efficacité du dispositif permet par exemple de réduire le diamètre extérieur de la cartouche d'environ 20mm (sur total typiquement de 90mm dans les solutions connues).

Cette simplification est possible par le fait que la cartouche (paroi ajourée) est directement intégrée dans le volume respirable de gaz à purifier. C'est-à-dire que le gaz à purifier est directement admis au travers des trous de la paroi ajourée dans la cartouche 4.

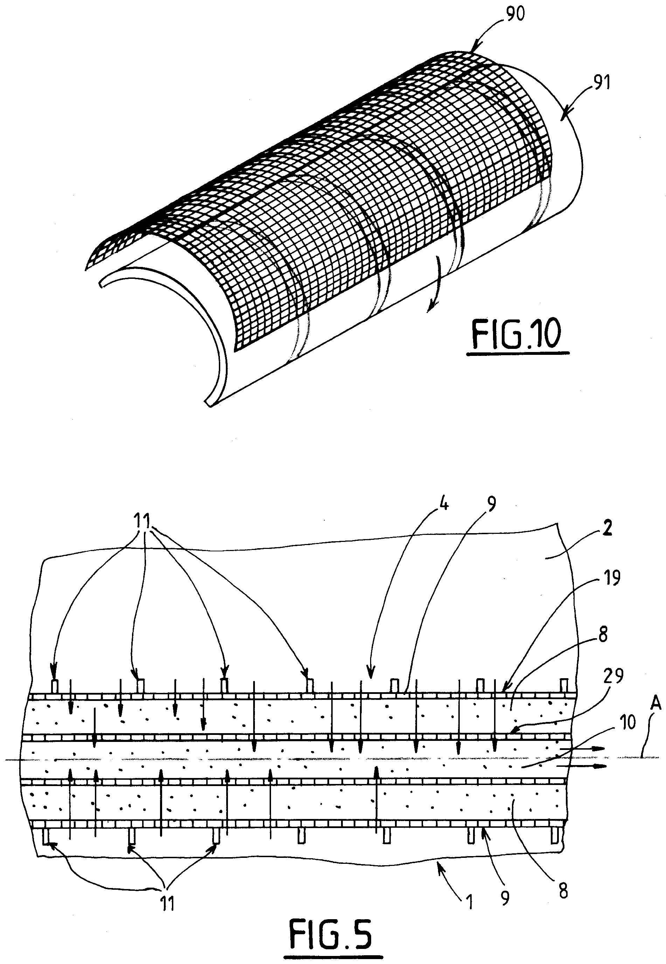

La figure 5 illustre un exemple de réalisation explicitant cette structure.

La cartouche 4 comprend une structure de tube 9 ajouré. Ce tube ajouré comprend une paroi extérieure 19, par exemple cylindrique, ajourée et une paroi 29 intérieure ajourée, par exemple cylindrique également et concentrique.

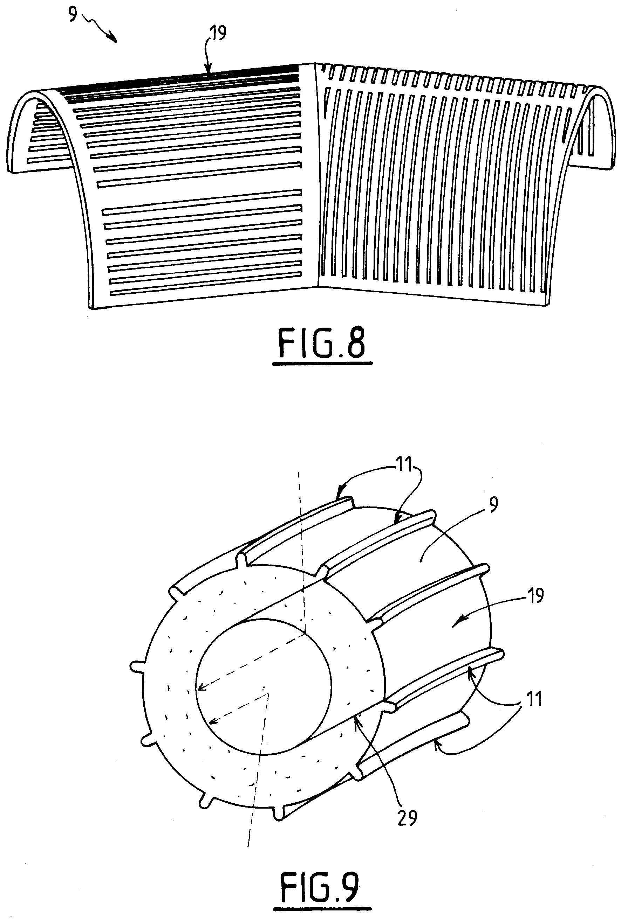

Le volume 8 de matériau à absorbant de CO2 est disposé et maintenu entre les parois intérieure 29 et extérieure 19. La paroi intérieure 29 communique avec un volume 10 central formant un chemin d'évacuation du gaz purifié (cf. également figure 9).

C'est-à-dire que la paroi 19 extérieur ajourée (munie de trous) communique directement avec le gaz dans l'enveloppe 2. Le trajet de gaz est schématisé la figure 5 via des flèches.

Pour éviter le cas échéant que l'enveloppe 2 souple ne vienne obturer les orifices d'entrée dans la cartouche 4, comme illustré à la figure 5, la surface périphérique du tube 9 ajouré peut comprendre une ou des saillies 11 qui s'étendent radialement vers l'extérieur du tube 9 ajouré par rapport à l'axe A.

Ces saillies 11 forment des « espaceurs » permettent d'éviter que l'enveloppe 2 souple ou la peau de l'utilisateur n'obstrue les orifices du tube 9 ajouré en empêchant un contact étanche contre la paroi 19 ajourée.

Ces saillies 11 permettent également d'éviter que la peau de l'utilisateur vienne en contact avec la grille contenant le matériau 8 adsorbant de CO2 car, généralement, la réaction de fixation du CO2 est exothermique (la température de la cartouche peut atteindre 40°C ce qui serait inconfortable)

Les saillies 11 s'étendent radialement par rapport à l'axe A longitudinal avec une hauteur comprise par exemple entre 1 mm et 5mm par rapport au reste de la surface périphérique du tube 9 ajouré.

Les saillies 11 sont de préférence réparties le long de la direction A longitudinale. Les saillies 11 peuvent être espacées les unes des selon la direction A longitudinale d'une distance comprise par exemple entre 1mm et 50mm.

Les saillies 11 peuvent être réparties sur au moins une partie et de préférence sur toute la circonférence du tube 9 ajouré.

Comme illustré à la figure 9, les saillies 11 peuvent former des nervures longitudinales parallèle à l'axe A longitudinal du tube 9 ajouré.

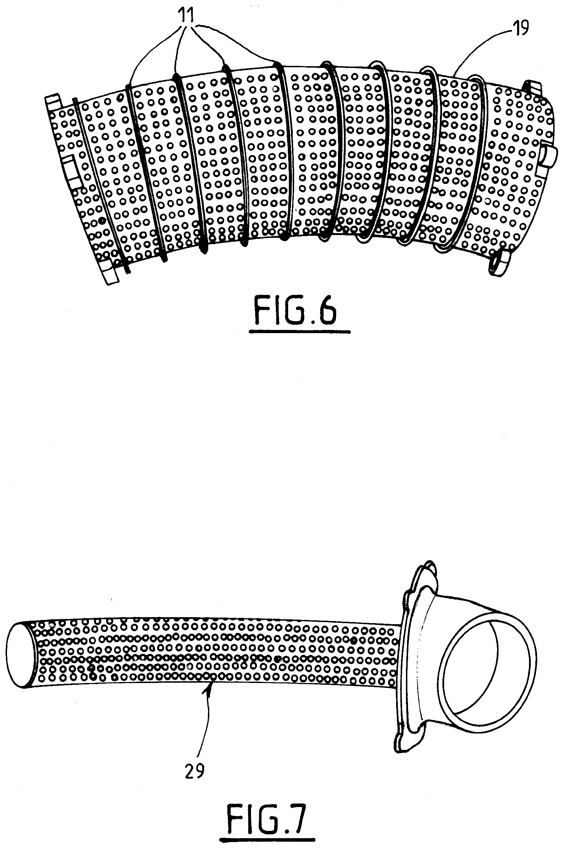

Comme illustré aux figure 5 ou 6, les saillies 11 peuvent former des nervures transversales (anneaux) perpendiculaires à l'axe A longitudinal du tube 9 ajouré.

Au lieu (ou en plus) de saillies 11 en forme de nervures, les saillies 11 peuvent être des tétons discrets répartis sur la paroi 19 ajourée.

Comme illustré aux figures 1, 6, 7 et 8, le réservoir 3 et/ou la cartouche 4 et donc le tube 9 ajouré peuvent être cintrés pour améliorer son intégration autour de la tête ou du cou de l'utilisateur. Ce cintrage peut être continu (cf. figure 6 et 7) ou obtenu par des portions rectilignes assemblées avec un angle (cf. figure 8).

De préférence cependant, la cartouche 4 filtrante est rectiligne (tube cylindrique dont la direction longitudinale A est rectiligne, c'est-à-dire non cintrée). De plus, de préférence le réservoir 3 d'oxygène est lui cintré (axe longitudinal cintré).

Classiquement le matériau 8 adsorbant le CO2 peut comprendre par exemple de la chaux sodée (Ca(OH)₂) ou de l'hydroxyde de lithium (LiOH). Ces matériaux réagissent toutes deux avec le CO₂ pour générer un composé carbonaté, de l'eau et de la chaleur.

Ces matériaux sont généralement utilisés sous forme de granules de différents diamètres conditionnés dans des cartouches radiales ou axiales mais parfois également sous forme de feuille (pate mise en forme) ou de matelas (granules contenues dans une enveloppe poreuse). Par exemple, le matériau 8 est logé dans le tube 9 ajouré et maintenu dans ce dernier via des bouchons montés aux extrémités du tube 9. Le bouchon de la cartouche radiale peut être un simple bouchon ou peut posséder une fonction de tassage du matériau 8 avec ressorts métallique ou pièce plastique déformable sous contrainte.

Les figures 3 et 4 illustrent schématiquement un agencement possible du système de génération de gaz respirable dans la cagoule 2.

Comme illustré à la figure 3, l'orifice 7 de sortie du réservoir 3 d'oxygène tubulaire et la sortie 6 de d'air filtré de la cartouche 4 de filtration peuvent être agencés relativement (en particulier concentriquement et coaxialement) pour former un dispositif venturi.

La figure 4 schématise les flux de gaz via des flèches. L'oxygène est fourni par le réservoir 3 d'oxygène au venturi qui génère alors une aspiration au travers de la cartouche 4 filtrante. Le mélange respirable enrichi en oxygène est délivré dans l'enveloppe 2 souple. Ce dispositif venturi permet ainsi d'utiliser l'énergie de détente du gaz fourni par le réservoir 3 sous pression pour créer un courant de recirculation au travers de la cartouche 4 filtrante (chaux sodée ou d'hydroxyde de lithium). De préférence, le venturi possède un ratio d'entrainement (débit aspiré/débit injecté) compris entre 10 et 20 sur la plage de débit injecté de 1 à 5 litre par minute par exemple.

Le tube 9 ajouré contenant l'entité 8 filtrante a de préférence une structure en forme de grille (plastique ou métallique ou autre).

Comme illustré aux figures 6 et 7 cette structure peut être une structure du type d'une grille rigide munie de trous circulaires.

Comme illustré à la figure 8 cette structure peut être une structure du type d'une grille rigide munie de trous en forme de rainures (longitudinales cf. partie de gauche et/ou transversales cf. partie de droite). Cette structure 9 peut être par exemple moulée.

La figure 10 illustre un autre exemple de réalisation du tube 9 ajouré. Celui-ci peut être composé d'une structure 91 de base comprenant un squelette recouvert d'une feuille 90 ajourée, notamment d'une feuille 90 plastique ajourée fixée sur la structure 91 de base. La structure 91 de base est réalisée par exemple par injection plastique. La structure 91 de base peut intégrer également la des saillies (« espaceurs »).

Par exemple, la fonction de grillage (paroi ajourée munie d'orifice de passage du gaz) peut être réalisée par une feuille 90 plastique ayant une structure proche de celle d'une moustiquaire (en polyamide avec des orifices de taille comprise entre 20 et 200µm).

Le tube 9 ajouré comprend de préférence une pluralité d'orifice prévus pour le passage de gaz, lesdits orifices ayant des surfaces comprises entre 3,14mm² et 600mm². Par exemple la valeur basse de 3,14mm² peut correspondre au cas où la cartouche comporterai une multitude d'orifices circulaires de rayon 1mm. De même, la valeur haute de 600 mm², peut correspondre au cas où la structure du tube ferait uniquement office de squelette pour une feuille du type moustiquaire apposée sur cette structure squelette. Ce squelette aurait par exemple des ouvertures (rectangulaires ou autre) de dimension pare exemple 6x100mm².

La géométrie radiale de la cartouche 4 permet de maximiser la longueur du volume de matériau 8 adsorbant de CO2 et donc permet de réduire la hauteur de lit d'entité poreuse à traverser. Ceci permet de diminuer le diamètre extérieur de la cartouche 4 en améliorant le temps de résidence et la perte de charge du gaz à purifier. Ceci est ainsi compatible avec l'encombrement de stockage visé pour ce type d'équipement dans des avions.

## Revendications

1. Cagoule de protection respiratoire comprenant une enveloppe (2) souple étanche destinée à être enfilée sur la tête d'un utilisateur, l'enveloppe souple étant munie d'une fenêtre (20) transparente et comprenant, dans sa partie inférieure, un élément (3, 4) de base rigide destiné à être disposé autour du cou de l'utilisateur, l'élément (3, 4) de base comprenant un réservoir (3) d'oxygène sous pression muni d'un orifice (7) de sortie calibré débouchant dans le volume interne de l'enveloppe (2) souple et un dispositif de captation d'au moins une partie du dioxyde de carbone expiré par l'utilisateur, pour épurer le gaz respirable au sein de la cagoule (2), **caractérisée en ce que** le dispositif de captation du dioxyde de carbone comprend une cartouche (4) d'adsorption du dioxyde de carbone à structure radiale comprenant un corps tubulaire s'étendant selon une direction (A) longitudinale et abritant un volume (8) de matériau absorbant du dioxyde de carbone, ladite structure de la cartouche (4) comprenant une tube (9) ajouré disposée autour du volume (8) de matériau absorbant et s'entendant selon la direction (A) longitudinale pour permettre le transite d'air à épurer radialement dans le volume (8) de matériau à absorbant, c'est-à-dire perpendiculairement à la direction (A) longitudinale, et **en ce que** qu'au moins une partie du tube (9) ajouré disposé autour du volume de matériau absorbant communique directement avec le volume interne de la cagoule (2), c'est-à-dire qu'au moins une partie du tube (9) ajouré n'est pas entouré d'une paroi extérieure supplémentaire pleine délimitant un volume diffuseur autour du tube (9) ajouré.

2. Cagoule selon la revendication 1, **caractérisée en ce que** le tube (9) ajouré comprend une paroi extérieure (19) ajourée et une paroi (29) intérieure ajourée, le volume (8) de matériau à absorbant étant disposé entre les parois intérieure (29) et extérieure (19).

3. Cagoule selon la revendication 2, **caractérisée en ce que** l'air épuré ayant transité dans le volume (8) de matériau absorbant est collecté dans un volume (10) collecteur d'air purifié, au moins une partie du volume (10) collecteur d'air purifié étant délimité par la paroi (29) intérieure du tube ajouré.

4. Cagoule selon l'une quelconque des revendications 1 à 3, **caractérisée en ce qu'**au moins une partie de la surface périphérique du tube (9) ajouré comprend des saillies (11) qui s'étendent radialement vers l'extérieur du tube (9) ajouré par rapport à l'axe (A) longitudinal, pour éviter que l'enveloppe (2) souple ou la peau de l'utilisateur n'obstrue les orifices du tube (9) ajouré.

5. Cagoule selon l'une la revendication 4, **caractérisée en ce que** les saillies (11) s'étendent radialement par rapport à l'axe (A) longitudinal avec une hauteur comprise entre 1 et 5mm par rapport au reste de la surface périphérique du tube (9) ajouré.

6. Cagoule selon la revendication 4 ou 5, **caractérisée en ce que** le tube (9) comprend des saillies (11) réparties le long de la direction (A) longitudinal, les saillies (11) étant espacées les unes des selon la direction (A) longitudinale d'une distance comprise entre 10mm et 50mm.

7. Cagoule selon l'une quelconque des revendications 4 à 6, **caractérisée en ce que** le tube (9) comprend des saillies (11) réparties sur au moins une partie et de préférence sur sa circonférence.

8. Cagoule selon l'une quelconque des revendications 4 à 7, **caractérisée en ce que** le tube (9) comprend des saillies (11) formant des nervures longitudinales parallèles à l'axe (A) longitudinal.

9. Cagoule selon l'une quelconque des revendications 4 à 8, **caractérisée en ce que** le tube (9) comprend des saillies (11) formant des nervures transversales perpendiculaires à l'axe (A) longitudinal.

10. Cagoule selon l'une quelconque des revendications 1 à 9, **caractérisée en ce que** le tube (9) ajouré comprend une pluralité d'orifice prévus pour le passage de gaz, lesdits orifices ayant des surfaces comprises entre 3,14mm² et 600mm².

11. Cagoule selon l'une quelconque des revendications 1 à 10, **caractérisée en ce que** le tube (9) ajouré comprend sur sa surface une pluralité d'orifice de forme circulaire et/ou oblongue et/ou trapézoïdales, lesdits orifices allongés étant parallèles ou perpendiculaires à la direction (A) longitudinale.

12. Cagoule selon l'une quelconque des revendications 1 à 11, **caractérisée en ce que** le tube (9) ajouré est composé d'une structure métallique ou plastique grillagée.

13. Cagoule selon l'une quelconque des revendications 1 à 12, **caractérisée en ce que** le tube (9) ajouré est composé d'une structure (91) de base comprenant un squelette recouvert d'une feuille (90) ajourée, notamment d'une feuille (90) plastique ajourée fixée sur la structure (91) de base.

14. Cagoule selon la revendication 13, **caractérisée en ce que** la feuille (90) ajourée est en matériau plastique et est collée ou surmoulée sur la structure (91) de base.

## Patentansprüche

1. Atemschutzhaube, umfassend eine dichte flexible Hülle (2), dazu bestimmt, um über den Kopf eines Benutzers gezogen zu werden, wobei die flexible Hülle mit einem transparenten Fenster (20) ausgestattet ist und in ihrem unteren Teil ein starres Basiselement (3, 4) umfasst, dazu bestimmt, um den Hals des Benutzers angeordnet zu sein, wobei das Basiselement (3, 4) einen Sauerstoffbehälter (3) unter Druck umfasst, der mit einer kalibrierten Ausgangsöffnung (7) ausgestattet ist, die in das innere Volumen der flexiblen Hülle (2) mündet, und eine Vorrichtung zum Einfangen von mindestens einem Teil des Kohlendioxids, das vom Benutzer ausgeatmet wird, um das atembare Gas innerhalb der Haube (2) zu reinigen, **dadurch gekennzeichnet, dass** die Vorrichtung zum Einfangen des Kohlendioxids eine Patrone (4) zur Adsorption des Kohlendioxids mit radialer Struktur umfasst, umfassend einen rohrförmigen Körper, der sich in eine Längsrichtung (A) erstreckt und ein Volumen (8) aus absorbierendem Material des Kohlendioxids beherbergt, wobei die Struktur der Patrone (4) ein durchbrochenes Rohr (9) umfasst, das um das Volumen (8) aus absorbierendem Material angeordnet ist und sich in die Längsrichtung (A) erstreckt, um die Übertragung von zu reinigender Luft radial in das Volumen (8) aus absorbierendem Material, d. h. senkrecht zur Längsrichtung (A) zu ermöglichen, und dadurch, dass mindestens ein Teil des durchbrochenen Rohrs (9), das um das Volumen aus absorbierendem Material angeordnet ist, direkt mit dem inneren Volumen der Haube (2) kommuniziert, d. h., dass mindestens ein Teil des durchbrochenen Rohrs (9) nicht von einer massiven zusätzlichen äußeren Wand umgeben ist, die ein Verteilervolumen um das durchbrochene Rohr (9) begrenzt.

2. Haube nach Anspruch 1, **dadurch gekennzeichnet, dass** das durchbrochene Rohr (9) eine durchbrochene äußere Wand (19) und eine durchbrochene innere Wand (29) umfasst, wobei das Volumen (8) aus absorbierendem Material zwischen der inneren (29) und der äußeren Wand (19) angeordnet ist.

3. Haube nach Anspruch 2, **dadurch gekennzeichnet, dass** die gereinigte Luft, die in das Volumen (8) aus absorbierendem Material übertragen wurde, in einem Sammelvolumen (10) von gereinigter Luft gesammelt wird, wobei mindestens ein Teil des Sammelvolumens (10) von gereinigter Luft von der inneren Wand (29) des durchbrochenen Rohrs begrenzt wird.

4. Haube nach einem der Ansprüche 1 bis 3, **dadurch gekennzeichnet, dass** mindestens ein Teil der peripheren Oberfläche des durchbrochenen Rohrs (9) Vorsprünge (11) umfasst, die sich radial hin zur Außenseite des durchbrochenen Rohrs (9) mit Bezug auf die Längsachse (A) erstrecken, um zu vermeiden, dass die flexible Hülle (2) oder die Haut des Benutzers die Öffnungen des durchbrochenen Rohrs (9) blockieren.

5. Haube nach Anspruch 4, **dadurch gekennzeichnet, dass** sich die Vorsprünge (11) radial mit Bezug auf die Längsachse (A) mit einer Höhe erstrecken, die zwischen 1 und 5 mm mit Bezug auf den Rest der peripheren Oberfläche des durchbrochenen Rohrs (9) enthalten ist.

6. Haube nach Anspruch 4 oder 5, **dadurch gekennzeichnet, dass** das Rohr (9) Vorsprünge (11) umfasst, die entlang der Längsrichtung (A) verteilt sind, wobei die Vorsprünge (11) voneinander in der Längsrichtung (A) um eine Distanz beabstandet sind, die zwischen 10 mm und 50 mm enthalten ist.

7. Haube nach einem der Ansprüche 4 bis 6, **dadurch gekennzeichnet, dass** das Rohr (9) Vorsprünge (11) umfasst, die auf mindestens einem Teil und vorzugsweise auf seinem Umfang verteilt sind.

8. Haube nach einem der Ansprüche 4 bis 7, **dadurch gekennzeichnet, dass** das Rohr (9) Vorsprünge (11) umfasst, die Längsrippen bilden, die parallel zur Längsachse (A) sind.

9. Haube nach einem der Ansprüche 4 bis 8, **dadurch gekennzeichnet, dass** das Rohr (9) Vorsprünge (11) umfasst, die Querrippen bilden, die senkrecht zur Längsachse (A) sind.

10. Haube nach einem der Ansprüche 1 bis 9, **dadurch gekennzeichnet, dass** das durchbrochene Rohr (9) eine Vielzahl von Öffnungen umfasst, die für den Durchgang von Gas vorgesehen sind, wobei die Öffnungen Oberflächen aufweisen, die zwischen 3,14 mm² und 600 mm² enthalten sind.

11. Haube nach einem der Ansprüche 1 bis 10, **dadurch gekennzeichnet, dass** das durchbrochene Rohr (9) auf seiner Oberfläche eine Vielzahl von kreisförmigen und/oder länglichen und/oder trapezförmigen Öffnungen umfasst, wobei die länglichen Öffnungen parallel oder senkrecht zur Längsrichtung (A) sind.

12. Haube nach einem der Ansprüche 1 bis 11, **dadurch gekennzeichnet, dass** das durchbrochene Rohr (9) aus einer vergitterten metallischen oder Kunststoffstruktur zusammengesetzt ist.

13. Haube nach einem der Ansprüche 1 bis 12, **dadurch gekennzeichnet, dass** das durchbrochene Rohr (9) aus einer Basisstruktur (91) zusammengesetzt ist, umfassend ein Gerüst, das mit einer durchbrochenen Folie (90), insbesondere einer durchbrochenen Kunststofffolie (90) bedeckt ist, die auf der Basisstruktur (91) fixiert ist.

14. Haube nach Anspruch 13, **dadurch gekennzeichnet, dass** die durchbrochene Folie (90) aus Kunststoffmaterial ist und auf die Basisstruktur (91) geklebt oder überformt ist.

## Claims

1. Respiratory protection hood comprising a flexible sealed shell (2) intended to be placed over the head of a user, the flexible shell being provided with a transparent screen (20) and comprising, in the lower portion thereof, a hard base element (3, 4) intended to be placed around the neck of the user, the base element (3, 4) comprising a pressurised oxygen tank (3) provided with a calibrated outlet orifice (7) opening into the inner volume of the flexible shell (2) and a device for collecting at least some of the carbon dioxide exhaled by the user, to purify the breathable gas inside the hood (2), **characterised in that** the carbon dioxide collection device comprises a cartridge (4) for the adsorption of the carbon dioxide with a radial structure comprising a tubular body extending along a longitudinal direction (A) and housing a volume (8) of material that absorbs carbon dioxide, said structure of the cartridge (4) comprising a tube (9) with holes arranged around the volume (8) of absorbent material and extending along the longitudinal direction (A) to make it possible for the air that is to be purified to travel radially in the volume (8) of absorbent material, i.e. perpendicular to the longitudinal direction (A), and **in that** at least one portion of the tube (9) with holes arranged around the volume of absorbent material communicates directly with the inner volume of the hood (2), i.e. at least one portion of the tube (9) with holes is not surrounded by an additional solid outer wall delimiting a diffuser volume around the tube (9) with holes.

2. Hood according to claim 1, **characterised in that** the tube (9) with holes comprises an outer wall (19) with holes and an inner wall (29) with holes, the volume (8) of absorbent material being arranged between the inner wall (29) and the outer wall (19).

3. Hood according to claim 2, **characterised in that** the purified air having travelled in the volume (8) of absorbent material is collected in a volume (10) for collecting purified air, at least one portion of the volume (10) for collecting purified air being delimited by the inner wall (29) of the tube with holes.

4. Hood according to any one of claims 1 to 3, **characterised in that** at least one portion of the peripheral surface of the tube (9) with holes comprises protrusions (11) that extend radially outwards from the tube (9) with holes with respect to the longitudinal axis (A), to prevent the flexible shell (2) or the skin of the user from blocking the orifices of the tube (9) with holes.

5. Hood according to claim 4, **characterised in that** the protrusions (11) extend radially with respect to the longitudinal axis (A) at a height of between 1 mm and 5 mm with respect to the remainder of the peripheral surface of the tube (9) with holes.

6. Hood according to claim 4 or 5, **characterised in that** the tube (9) comprises protrusions (11) distributed along the longitudinal direction (A), the protrusions (11) being spaced from one another in the longitudinal direction (A) by a distance of between 10 mm and 50 mm.

7. Hood according to any one of claims 4 to 6, **characterised in that** the tube (9) comprises protrusions (11) distributed on at least one portion and preferably around the circumference thereof.

8. Hood according to any one of claims 4 to 7, **characterised in that** the tube (9) comprises protrusions (11) forming longitudinal ribs parallel to the longitudinal axis (A).

9. Hood according to any one of claims 4 to 8, **characterised in that** the tube (9) comprises protrusions (11) forming transversal ribs perpendicular to the longitudinal axis (A).

10. Hood according to any one of claims 1 to 9, **characterised in that** the tube (9) with holes comprises a plurality of orifices for the passage of gases, said orifices having surfaces of between 3.14 mm² and 600 mm².

11. Hood according to any one of claims 1 to 10, **characterised in that** the tube (9) with holes comprises, on the surface thereof, a plurality of orifices with a circular and/or oblong and/or trapezoidal shape, said elongated orifices being parallel or perpendicular to the longitudinal direction (A).

12. Hood according to any one of claims 1 to 11, **characterised in that** the tube (9) with holes is made of a metal structure or of plastic with wiring.

13. Hood according to any one of claims 1 to 12, **characterised in that** the tube (9) with holes is made of a base structure (91) comprising a skeleton covered with a sheet (90) with holes, in particular a plastic sheet (90) with holes secured to the base structure (91).

14. Hood according to claim 13, **characterised in that** the sheet (90) with holes is made of a plastic material and is glued or overmoulded on the base structure (91).
